# EUROPEAN PATENT SPECIFICATION

(11) **EP 3 532 635 B1**
(45) Date of publication and mention of the grant of the patent: **09.06.2021**
(21) Application number: 17787432.8
(22) Date of filing: 24.10.2017
(51) Int. Cl.: C12Q 1/68

(54) **BARCODED CIRCULAR LIBRARY CONSTRUCTION FOR IDENTIFICATION OF CHIMERIC PRODUCTS**
AUFBAU EINER STRICHCODIERTEN KREISFÖRMIGEN BIBLIOTHEK ZUR IDENTIFIZIERUNG VON CHIMÄREN PRODUKTEN
CONSTRUCTION DE BIBLIOTHÈQUE CIRCULAIRE À CODE-BARRES POUR L'IDENTIFICATION DE PRODUITS CHIMÉRIQUES

(30) Priority: 31.10.2016 US 201662415245 P
(43) Date of publication of application: 04.09.2019
(73) Proprietor: F. Hoffmann-La Roche AG, 4070 Basel (CH); Roche Diagnostics GmbH, 68305 Mannheim (DE)
(72) Inventor: GUETTOUCHE, Toumy, Pleasanton, California 94588 (US); RICHARDSON, Aaron, Pleasanton, California 94588 (US)
(74) Representative: Hildebrandt, Martin K. E.
(86) International application number: PCT/EP2017/077111
(87) International publication number: WO 2018/077847

(56) References cited:
- WO-A1-2007/018601
- MARK T. GREGORY ET AL: "Targeted single molecule mutation detection with massively parallel sequencing", NUCLEIC ACIDS RESEARCH, 17 September 2015 (2015-09-17), page gkv915, XP055241840, GB ISSN: 0305-1048, DOI: 10.1093/nar/gkv915

## Description

### FIELD OF THE INVENTION

The invention relates to the field of nucleic acid sequencing. More specifically, the invention relates to the field of creating barcoded template libraries for nucleic acid sequencing.

### BACKGROUND OF THE INVENTION

The current generation of nucleic acid sequencing methods utilizes libraries of target molecules from which each individual molecule is sequenced. Each molecule in the library comprises a target sequence to be analyzed conjugated to artificial sequences necessary for the chosen sequencing method and sequencing instrument. The artificial sequences commonly include barcodes, short sequences of nucleotides used to uniquely mark an individual molecule or a group of molecules. M. T. GREGORY at al. (Nucleic Acids Research, 2016, Vol. 44, No. 3 e22) disclose methods for targeted single molecule mutation detection with massively parallel sequencing. In contrast, WO 2007/018601 A1 discloses compositions and methods for processing and amplification of DNA, including using multiple enzymes in a single reaction.

Unique molecular barcodes have multiple uses. Marking and tracing each individual nucleic acid molecule enables detection of extremely rare sequences, *e.g*., circulating tumor DNA (ctDNA) present in trace amounts in patient's blood and used for non-invasive early detection and precise monitoring of cancer (*See* Newman, A., et al., (2014) An ultrasensitive method for quantitating circulating tumor DNA with broad patient coverage, Nature Medicine doi:10.1038/nm.3519.) The entire progeny of a single target molecule is marked with the same barcode and forms a barcoded family. Therefore barcodes may be used for error correction. A variation in the sequence not shared by all members of the barcoded family is discarded as an artifact and not a true mutation. Barcodes can also be used for positional deduplication and target quantification, as the entire family represents a single molecule in the original sample *(See* Newman, A., et al., (2016) Integrated digital error suppression for improved detection of circulating tumor DNA, Nature Biotechnology 34:547).

The barcode-enabled error correction has greatly enhanced the sensitivity of sequencing assays. Sequencing artifacts such as polymerase errors are no longer a barrier to detecting rare point mutations. At the same time, barcodes have not been as beneficial for detecting translocations (gene fusions), another common type of mutation in human malignancies. See F. Mertens, et al. (2015) The emerging complexity of gene fusions in cancer, Nat. Rev. Cancer 15:371; F. Mitelman, et al. (2007) The impact of translocations and gene fusions on cancer causation, Nat. Rev. Cancer 7:233. Since barcodes are typically randomly ligated to both ends of a target molecule, it is unknown which 5' barcodes originally are associated with which 3' barcodes. This poses a problem during the amplification step of library preparation, as chimeric molecules are produced via template switching in PCR. An artificially produced chimeric molecule present in the sequencing library is indistinguishable from an authentic gene fusion that may have been present in the original sample. This directly limits the capacity to detect low-frequency gene fusions, which can be important driver mutations in cancer. A barcode-based method is needed to trace and eliminate artificial gene fusions to enable detection of true mutations.

### SUMMARY OF THE INVENTION

In some embodiments, the invention is a method of making a library of target nucleic acid molecules from a sample comprising a plurality of target molecules, the method comprising for substantially each target molecule: ligating a single adaptor to a target molecule forming a circular molecule, wherein the adaptor comprises two barcodes, two primer binding sites situated between the two barcodes, wherein the primers annealing to the binding sites are facing away from each other, and at least one modified nucleotide effecting a strand synthesis termination by a nucleic acid polymerase situated between the two primer binding sites; annealing a forward primer complementary to the adaptor to one strand of the target molecule; extending the forward primer up to the modified nucleotide, thereby producing a first strand; annealing a reverse primer complementary to the adaptor to the first strand; extending the first primer, thereby producing the second strand and a double-stranded molecule comprising the first strand sand the second strand wherein the two barcodes are flanking the target sequence. In some embodiments, at least one of the forward and the reverse primer comprises a 5'-flap sequence not complementary to the adaptor and comprising an additional primer binding site. Then the method further comprises a step of annealing an additional primer to the sequence complementary to the flap sequence in the forward primer and extending the additional primer thereby producing a double-stranded molecule comprising two additional primer sites and the two barcodes flanking the target sequence. In some embodiments, the target molecule and the adaptor are single-stranded. In other embodiments, the target molecule and the adaptor are double-stranded and the circular molecule is at least partially denatured primer to annealing of the primer. In some embodiments the barcode is a nucleotide sequence 4-20 bases long. The modified nucleotide effecting a strand synthesis termination by a nucleic acid polymerase may be selected from abasic nucleotides, nucleotides with protein side groups, synthetic nucleotide AraC (cytarabine) or deoxyuracil, isoguanine, 5-methylisocytosine, ethylene glycol spacers, nucleotides with bulky analogues such as fluorophores, or unnatural base pair (UBP) "d5SICS-dNaM" nucleic acid analogues. The ligation may be selected from overhang ligation, T-A ligation, blunt-end ligation and topoisomerase catalyzed ligation. In some embodiments, the adaptor has a photocleavable linker on one end. In these embodiments, the linker is ligated on one end and exposed to UV light to enable ligation on the other end. In some embodiments, the additional primers are sequencing primers.

In some embodiments, the invention is a library of target nucleic acid molecules wherein each molecule is a circular molecule comprising a target sequence and an adaptor linking the ends of the target sequence, the adaptor comprising: two barcodes; two primer binding sites situated between the two barcodes, wherein the primers annealing to the binding sites are facing away from each other; at least one modified nucleotide effecting a strand synthesis termination by a nucleic acid polymerase situated between the two primer binding sites. In some embodiments, the barcode is a nucleotide sequence 4-20 bases long. The modified nucleotide effecting a strand synthesis termination by a nucleic acid polymerase may be selected from abasic nucleotides, nucleotides with protein side groups, synthetic nucleotide AraC (cytarabine) or deoxyuracil, isoguanine, 5-methylisocytosine, ethylene glycol spacers, nucleotides with bulky analogues such as fluorophores, or unnatural base pair (UBP) "d5SICS-dNaM" nucleic acid analogues.

In other embodiments, the invention is a method of sequencing target nucleic acids in a sample comprising a plurality of target molecules, the method comprising: creating a library of target nucleic acid molecules from the sample by ligating a single double-stranded adaptor to substantially each double-stranded target molecule forming a double stranded circular molecule, wherein the adaptor comprises two barcodes, two primer binding sites situated between the two barcodes, wherein the primers annealing to the binding sites are facing away from each other, and at least one modified nucleotide effecting a strand synthesis termination by a nucleic acid polymerase situated between the two primer binding sites; denaturing at least a portion of the double-stranded circular target molecule; annealing a forward primer complementary to the adaptor to one strand of the target molecule; extending the forward primer up to the modified nucleotide, thereby producing a first strand; annealing a reverse primer complementary to the adaptor to the first strand; extending the first primer, thereby producing the second strand and a double-stranded molecule comprising the first strand and the second strand wherein the two barcodes are flanking the target sequence; amplifying the double stranded molecule; and sequencing the amplified products of the double-stranded molecule. In some embodiments, at least one of the forward and the reverse primer comprises a 5'-flap sequence not complementary to the adaptor and comprising an additional primer binding site. In some embodiments, the method further comprises after extending the first primer, annealing an additional primer to the sequence complementary to the flap sequence in the forward primer and extending the additional primer thereby producing a double-stranded molecule comprising two additional primer sites and the two barcodes flanking the target sequence. In some embodiments, amplifying or sequencing may be performed with the additional primers.

### BRIEF DESCRIPTION OF THE DRAWINGS

Figure 1 is a diagram of a single-stranded barcoded library molecule according to the invention.
Figure 2 is a diagram of the first strand synthesis initiation with a forward primer.
Figure 3 is a diagram of the first strand synthesis and termination.
Figure 4 is a diagram of the completed first strand.
Figure 5 is a diagram of the second strand synthesis initiation with a reverse primer using the first strand as template.
Figure 6 is a diagram of the completed second strand.
Figure 7 is a diagram of the next round of the first strand synthesis initiation with a forward primer using the second strand as template.
Figure 8 is a diagram of a completed sequencing template molecule.

### DETAILED DESCRIPTION OF THE INVENTION

### Definitions

The following definitions aid in understanding of this disclosure.

The term "sample" refers to any composition containing or presumed to contain target nucleic acid. This includes a sample of tissue or fluid isolated from an individual for example, skin, plasma, serum, spinal fluid, lymph fluid, synovial fluid, urine, tears, blood cells, organs and tumors, and also to samples of *in vitro* cultures established from cells taken from an individual patient or from a model organism, including the formalin-fixed paraffin embedded tissues (FFPET) and nucleic acids isolated therefrom. A sample may also include cell-free material, such as cell-free blood fraction that contains cell-free DNA (cfDNA) or circulating tumor DNA (ctDNA).

A term "nucleic acid" refers to polymers of nucleotides (e.g., ribonucleotides and deoxyribonucleotides, both natural and non-natural) including DNA, RNA, and their subcategories, such as cDNA, mRNA, etc. A nucleic acid may be single-stranded or double-stranded and will generally contain 5'-3' phosphodiester bonds, although in some cases, nucleotide analogs may have other linkages. Nucleic acids may include naturally occurring bases (adenosine, guanosine, cytosine, uracil and thymidine) as well as non-natural bases. Some examples of non-natural bases include those described in, *e.g.,* Seela et al., (1999) Helv. Chim. Acta 82:1640. The non-natural bases may have a particular function, *e.g*., increasing the stability of the nucleic acid duplex, inhibiting nuclease digestion or blocking primer extension or strand polymerization.

The terms "polynucleotide" and "oligonucleotide" are used interchangeably. Polynucleotide is a single-stranded or a double-stranded nucleic acid. Oligonucleotide is a term sometimes used to describe a shorter polynucleotide. An oligonucleotide may be comprised of at least 6 nucleotides or about 15-30 nucleotides. Oligonucleotides are prepared by any suitable method known in the art, for example, by a method involving direct chemical synthesis as described in Narang et al. (1979) Meth. Enzymol. 68:90-99; Brown et al. (1979) Meth. Enzymol. 68:109-151; Beaucage et al. (1981) Tetrahedron Lett. 22:1859-1862; Matteucci et al. (1981) J. Am. Chem. Soc. 103:3185-3191.

The term "primer" refers to a single-stranded oligonucleotide which hybridizes with a sequence in a target nucleic acid ("primer binding site") and is capable of acting as a point of initiation of synthesis along a complementary strand of nucleic acid under conditions suitable for such synthesis. The primer binding site can be unique to each target or can be added to all targets ("universal priming site" or "universal primer binding site").

The term "adaptor" means a nucleotide sequence that may be added to another sequence so as to import additional properties to that sequence. An adaptor is typically an oligonucleotide that can be single- or double-stranded, or may have both a single-stranded portion and a double-stranded portion. An adaptor may contain sequences such as barcodes and universal primer or probe sites.

The term "ligation" refers to a condensation reaction joining two nucleic acid strands wherein a 5'-phosphate group of one molecule reacts with the 3'-hydroxyl group of another molecule. Ligation is typically an enzymatic reaction catalyzed by a ligase or a topoisomerase. Ligation may join two single strands to create one single-stranded molecule. Ligation may also join two strands each belonging to a double-stranded molecule thus joining two double-stranded molecules. Ligation may also join both strands of a double-stranded molecule to both strands of another double-stranded molecule thus joining two double-stranded molecules. Ligation may also join two ends of a strand within a double-stranded molecule thus repairing a nick in the double-stranded molecule.

The term "barcode" refers to a nucleic acid sequence that can be detected and identified. Barcodes can be incorporated into various nucleic acids. Barcodes are sufficiently long *e.g.,* 2, 5, 10 nucleotides, so that in a sample, the nucleic acids incorporating the barcodes can be distinguished or grouped according to the barcodes.

The terms "multiplex identifier" and "MID" refer to a barcode that identifies a source of a target nucleic acids (*e.g.,* a sample from which the nucleic acid is derived, which is needed when nucleic acids from multiple samples are combined). All or substantially all the target nucleic acids from the same sample will share the same MID. Target nucleic acids from different sources or samples can be mixed and sequenced simultaneously. Using the MIDs the sequence reads can be assigned to individual samples from which the target nucleic acids originated.

The terms "unique molecular identifier" and "UID" refer to a barcode that identifies a nucleic acid to which it is attached. All or substantially all the target nucleic acids from the same sample will have different UIDs. All or substantially all of the progeny (*e.g.,* amplicons) derived from the same original target nucleic acid will share the same UID.

The term "universal primer" and "universal priming binding site" or "universal priming site" refer to a primer and primer binding site present in (typically, *in vitro* added to) different target nucleic acids. For example, the universal priming site may be included in an adaptor ligated to the plurality of target nucleic acids. The universal priming site may also be a part of target-specific (non-universal) primers, for example by being added to the 5'-end of a target-specific primer. The universal primer can bind to and direct primer extension from the universal priming site.

As used herein, the terms "target sequence", "target nucleic acid" or "target" refer to a portion of the nucleic acid sequence in the sample which is to be detected or analyzed. The term target includes all variants of the target sequence, *e.g*., one or more mutant variants and the wild type variant.

The term "sequencing" refers to any method of determining the sequence of nucleotides in the target nucleic acid.

Nucleic acid sequencing is rapidly expanding into clinical practice. The current sequencing technologies employ single molecule sequencing and allow detection of extremely rare targets. For example, nucleic acid sequencing has been used to detect rare tumor DNA shed into a patient's bloodstream. Detecting individual molecules typically requires molecular barcodes such as described in U.S. Patent Nos. 7,393,665, 8,168,385, 8,481,292, 8,685,678, and 8,722,368. A unique molecular barcode is a short artificial sequence added to each molecule in the patient's sample typically during the earliest steps of *in vitro* manipulations. The barcode marks the molecule and its progeny. The unique molecular barcode (UID) has multiple uses. Barcodes allow tracking each individual nucleic acid molecule in the sample to assess, *e.g*., the presence and amount of circulating tumor DNA (ctDNA) molecules in a patient's blood in order to detect and monitor cancer without a biopsy (Newman, A., et al., (2014) An ultrasensitive method for quantitating circulating tumor DNA with broad patient coverage, Nature Medicine doi:10.1038/nm.3519).

Unique molecular barcodes can also be used for sequencing error correction. The entire progeny of a single target molecule is marked with the same barcode and forms a barcoded family. A variation in the sequence not shared by all members of the barcoded family is discarded as an artifact and not a true mutation. Barcodes can also be used for positional deduplication and target quantification, as the entire family represents a single molecule in the original sample (Newman, A., et al., (2016) Integrated digital error suppression for improved detection of circulating tumor DNA, Nature Biotechnology 34:547).

The barcode-enabled error correction has greatly enhanced the sensitivity of sequencing assays. Sequencing artifacts such as polymerase errors are no longer a barrier to detecting rare point mutations. At the same time, barcodes have not been as beneficial for detecting translocations (gene fusions), another common type of mutation in human malignancies. See F. Mertens, et al. (2015) The emerging complexity of gene fusions in cancer, Nat. Rev. Cancer 15:371; F. Mitelman, et al. (2007) The impact of translocations and gene fusions on cancer causation, Nat. Rev. Cancer 7:233. Since barcodes are typically randomly ligated to both end of a target molecule, it is unknown which 5' barcodes originally are associated with which 3' barcodes. This poses a problem during the amplification step of library preparation, as chimeric molecules are produced via template switching in PCR. An artificially produced chimeric molecule present in the sequencing library is indistinguishable from an authentic gene fusion that may have been present in the original sample. This directly limits the capacity to detect low-frequency gene fusions, which can be important driver mutations in cancer. A barcode-based method is needed to trace and eliminate artificial gene fusions to enable detection of true mutations.

In some embodiments, the invention is a library of barcoded circular molecules for nucleic acid sequencing.

In some embodiments, the invention is a method of sequencing nucleic acids via creation of a library of circular barcoded nucleic acid molecules.

In some embodiments, the invention is a method of error correction in nucleic acid sequencing that utilizes barcodes to authenticate gene fusion molecules present in the original sample. In variations of this embodiment, the invention is a method of error correction in nucleic acid sequencing that utilizes barcodes to eliminate artificial gene fusion molecules not present in the original sample but generated during the steps of nucleic acid sequencing.

The present invention comprises detecting a target nucleic acid in a sample by nucleic acid sequencing. Multiple nucleic acids, including all the nucleic acids in a sample may be detected using the method and compositions described herein. In some embodiments, the sample is derived from a subject or a patient. In some embodiments the sample may comprise a fragment of a solid tissue or a solid tumor derived from the subject or the patient, *e.g*., by biopsy. The sample may also comprise body fluids (e.g., urine, sputum, serum, plasma or lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, or fecal samples). The sample may comprise whole blood or blood fractions where normal or tumor cells may be present. In some embodiments, the sample, especially a liquid sample may comprise cell-free material such as cell-free DNA or RNA including cell-free tumor DNA or tumor RNA. In some embodiments, the sample is a cell-free sample, *e.g*., cell-free blood-derived sample where cell-free tumor DNA or tumor RNA are present. In other embodiments, the sample is a cultured sample, *e.g*., a culture or culture supernatant containing or suspected to contain nucleic acids derived from the cells in the culture or from an infectious agent present in the culture. In some embodiments, the infectious agent is a bacterium, a protozoan, a virus or a mycoplasma.

A target nucleic acid is the nucleic acid of interest that may be present in the sample. In some embodiments, the target nucleic acid is a gene or a gene fragment. In some embodiments, all the genes, gene fragments and intergenic regions (entire genome) constitute target nucleic acids. In some embodiments, only a portion of the genome, *e.g*., only coding regions of the genome (exome) constitute target nucleic acids. In some embodiments, the target nucleic acid contains a locus of a genetic variant, *e.g*., a polymorphism, including a single nucleotide polymorphism or variant (SNP of SNV), or a genetic rearrangement resulting *e.g*., in a gene fusion. In some embodiments, the target nucleic acid comprises a biomarker, *i.e.,* a gene whose variants are associated with a disease or condition. In other embodiments, the target nucleic acid is characteristic of a particular organism and aids in identification of the organism or a characteristic of the pathogenic organism such as drug sensitivity or drug resistance. In yet other embodiments, the target nucleic acid is characteristic of a human subject, *e.g*., the HLA or KIR sequence defining the subject's unique HLA or KIR genotype.

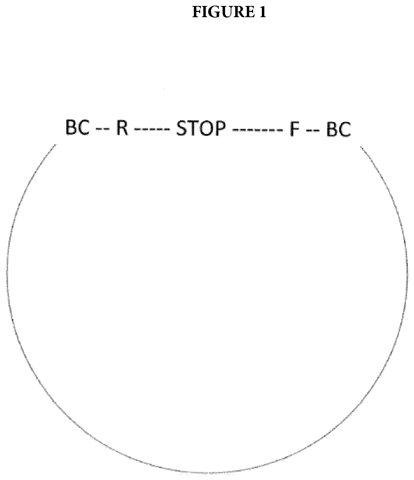

In an embodiment of the invention, one or a plurality of target nucleic acids is converted into the template configuration of the invention. In some embodiments, the target nucleic acid occurs in nature in a single-stranded form (e.g., RNA, including mRNA, microRNA, viral RNA; or single-stranded viral DNA). In other embodiments, the target nucleic acid occurs in nature in a double-stranded form. One of skill in the art would recognize that the method of the invention has multiple embodiments. A single stranded target nucleic acid can be converted into the structure of the invention as shown on Figure 1. A double stranded target nucleic acid can be converted into a double stranded structure where each strand is as depicted in Figure 1. Alternatively, the single-stranded target nucleic acid can be first converted into double-stranded form prior to following the remaining steps of the method disclosed herein. Longer target nucleic acids may be fragmented although in some applications longer target nucleic acids may be desired to achieve a longer read. In some embodiments, the target nucleic acid is naturally fragmented, *e.g*., circulating cell-free DNA (cfDNA) or chemically degraded DNA such as the one founds in preserved samples.

The present invention comprises the use of one adaptor molecule to be ligated to both ends of one target nucleic acid thus forming a circular molecule. In some embodiments, the adaptor is a single strand ligated to a single stranded target nucleic acid molecule. In some embodiments, ligating single-stranded nucleic acids is performed using splint oligonucleotides *see e.g.,* U.S. Application Pub. No. 20120003657. In other embodiments, ligating single-stranded nucleic acids or partially single-stranded nucleic acids is performed using 5'- and 3'- end single stranded regions (overhangs) *see e.g*., U.S. Application Pub. No. 20140193860. In other embodiments, the adaptor is a double stranded molecule ligated to a double stranded target nucleic acid molecule. Ligation of double stranded molecules is well known in the art *(See* Green M., and Sambrook, J., Molecular Cloning, 2012 CSHL Press), and improvements on the general method are described herein. In some embodiments, the double stranded ligation is a blunt-end ligation. In other embodiments, the double stranded ligation is a T-A ligation or other overhang ligation. In other embodiments, the double stranded ligation is driven by topoisomerase.

In some embodiments, the double-stranded adaptor has a photo-cleavable spacer on one of the two ends. In this set-up, only one end could ligate to a library molecule in a ligation reaction. Following a period of ligation, the reaction would be exposed to long wavelength UV (~350 nm), cleaving off the photo-cleavable spacer, and leaving a phosphorylated 5'-end of the adaptor. The ligation reaction may continue to form a circular template. In some embodiments, after the photocleavage, the reaction is diluted to reduce template concentration and facilitate self-ligation into circles. In this embodiment, the ligation results in reduced artifact formation (*e.g.,* DNA1 - adaptor - DNA2 or adaptor1 - DNA - adaptor2) and greater recovery of target nucleic acid molecules (greater GE (genome equivalent)) recovery.

In some embodiments, the adaptor molecules are *in vitro* synthesized artificial sequences. In other embodiments, the adaptor molecules are *in vitro* synthesized naturally-occurring sequences. In yet other embodiments, the adaptor molecules are isolated naturally occurring molecules or isolated non naturally-occurring molecules.

In some embodiments, the adaptor comprises one or more barcodes. A barcode can be a multiplex sample ID (MID) used to identify the source of the sample where samples are mixed (multiplexed). The barcode may also serve as a unique molecular ID (UID) used to identify each original molecule and its progeny. The barcode may also be a combination of a UID and an MID. In some embodiments, a single barcode is used as both UID and MID.

In some embodiments, each barcode comprises a predefined sequence. In other embodiments, the barcode comprises a random sequence. Barcodes can be 1-20 nucleotides long.

In embodiments of the present invention, the library molecules contain at least two barcodes included in the adaptor that is ligated to the target nucleic acid. In some embodiments of the invention, the barcodes are between about 4-20 bases long so that between 96 and 384 different adaptors, each with a different pair of identical barcodes are added to a human genomic sample. A person of ordinary skill would recognize that the number of barcodes depends on the complexity of the sample (*i.e.,* expected number of unique target molecules) and would be able to create a suitable number of barcodes for each experiment.

In some embodiment, the invention comprises a pool of adaptors for creating a library of circular barcoded molecules. The adaptors within the pool have a pair of identical barcodes that are at least 1 or at least 3 edit distance apart from other barcodes in the pool. One of skilled in the art would be able to determine what edit distance is optimal for a particular experiment based on typical error rates of a sequencing technology. Generally, greater edit distance means that fewer barcodes can be used in one pool. However, if the sequencing technology or a manufacturing process has a high error rate, greater edit distance will be required. For example, oligonucleotide manufacturing process used to make adaptors may have a high error rate. Similarly, a nucleic acid polymerase used in DNA amplification or primer extension in the sequencing-by-synthesis workflow can have a high error rate. These error rates would require increasing edit distance among the barcodes in adaptors of the pool. Conversely, improving the accuracy of each of the methods mentioned above will allow decreasing edit distance among the barcodes in adaptors of the pool.

In some embodiments, the invention comprises an article of manufacture represented by a single vial containing the entire pool of adaptors. Alternatively, an article of manufacture can comprise a kit where one or more adaptors of the pool are present in separate vials.

The adaptor further comprises a primer binding site for at least one universal primer. If two primer binding sites are present, the two primers are facing in opposite directions. One of skill in the art will recognize that a double stranded adaptor sequence will have a primer binding site on one or both strands. The primer binding site is a sequence complementary to the primer to which primer can bind and facilitate strand elongation. At the same time, one of skill in the art will recognize that a single stranded adaptor sequence will have a primer binding site for the first primer and a sequence identical to the second primer.

In some embodiments, the adaptor has two primer binding sites facing in the opposite direction so as to enable copying of each strand and subsequent PCR amplification of the two strands. In other embodiments, the adaptor has only one primer binding site to enable coping of only one strand. In some embodiments, more than one round of copying is desired. Several rounds can be performed with the same primer or different primers. The adaptor can have several primer binding sites, *e.g*., second primer binding site internal to the first primer binding site. Alternatively, one or both of the forward and the reverse primer may comprise a 5'-flap sequence not complementary to the adaptor and comprising an additional primer binding site.

In some embodiments, the adaptor comprises a nucleic acid synthesis termination (STOP) site. The site comprises one or more nucleotides or nucleotide analogs that are not bypassable by a nucleic acid polymerase. In some embodiments, the STOP site is one or more nucleotides and nucleotide analogues selected from abasic nucleotides, nucleotides with protein side groups, synthetic nucleotide AraC (cytarabine) or deoxyuracil, isoguanine, 5-methylisocytosine, ethylene glycol spacers, nucleotides with bulky analogues such as fluorophores, or unnatural base pair (UBP) "d5SICS-dNaM" nucleic acid analogues (*See* Malyshev, D., et al., (2012) Efficient and sequence-independent replication of DNA containing a third base pair establishes a functional six-letter genetic alphabet. P.N.A.S. 109 (30): 12005.) One of skill in the art would appreciate that a terminator nucleotide may be specific for a particular nucleic acid polymerase while other nucleic acid polymerases may be able to bypass the same terminator. For example, alkylated deoxyguanines (N7 and N2) are commonly synthesis terminators for *Taq* DNA polymerase. *See* Ponti, M., et al. (1991) Measurement of the sequence specificity of covalent DNA modification by antineoplastic agents using Taq DNA polymerase, Nucl. Acids Res. 19:2929. Likewise, deoxyuracil in DNA causes stall of some polymerases while others bypass it. Wardle, J., et al. (2008) Uracil recognition by replicative DNA polymerases is limited to the archaea, not occurring with bacteria and eukarya, Nucl. Acids Res. 36 (3):705-711.

In some embodiments, the invention utilizes enzymes. The enzymes may include a DNA polymerase (including sequencing polymerase), a DNA ligase and a terminal transferase.

In some embodiments, the DNA polymerase is a high-fidelity DNA polymerase that efficiently terminates synthesis at an unusual base, *i.e.,* the STOP site used in the present invention. Examples of high fidelity polymerases are archaeal polymerases such as *Pfu* (from *Pyrococcus furiosus*)*.* In other embodiments, *Taq* polymerase is used. In some embodiments, the polymerase possesses a 3'-5' exonuclease activity. In other embodiments, the polymerase does not have a strand displacement activity.

In some embodiments, the invention also utilizes a DNA ligase. In some embodiments, T4 DNA ligase or *E. coli* DNA ligase is used.

In some embodiments, the invention also utilizes a template-independent DNA polymerase, *e.g*., a terminal transferase or a DNA polymerase with the activity of adding one or more nucleotides in a template-independent manner. In some embodiments, the invention uses a mammalian terminal transferase or a *Taq* polymerase.

In some embodiments, the invention comprises an amplification step. This step can involve linear or exponential amplification, *e.g*., PCR. Amplification may be isothermal or involve thermocycling. In some embodiments, the amplification is exponential and involves PCR. Universal primers are used, *i.e*., a single pair of primers hybridizes to a binding site in the adaptor. All molecules in the library having the same adaptor can be amplified with the same set of primers. Because PCR with universal primers has reduced sequence bias, the number of amplification cycles need not be limited. The number of amplification cycles where universal primers are used can be low but also can be 10, 20 or as high as about 30 or more cycles, depending on the amount of product needed for the subsequent steps.

### Sequencing

The library of circular barcoded molecules and the linear amplicons generated from the library can be subjected to nucleic acid sequencing. Sequencing can be performed by any method known in the art. Especially advantageous is the high-throughput single molecule sequencing. Examples of such technologies include the Illumina HiSeq platform (Illumina, San Diego, Cal.), Ion Torrent platform (Life Technologies, Grand Island, NY), Pacific BioSciences platform utilizing the SMRT (Pacific Biosciences, Menlo Park, Cal.) or a platform utilizing nanopore technology such as those manufactured by Oxford Nanopore Technologies (Oxford, UK) or Roche Genia (Santa Clara, Cal.) and any other presently existing or future DNA sequencing technology that does or does not involve sequencing by synthesis. The sequencing step may utilize platform-specific sequencing primers. Binding sites for these primers may be introduced in 5'-portions of the amplification primers used in the amplification step. If no primer sites are present in the library of barcoded molecules, an additional short amplification step introducing such binding sites may be performed.

In some embodiments, the sequencing step involves sequence analysis. In some embodiments, the analysis includes a step of sequence aligning. In some embodiments, aligning is used to determine a consensus sequence from a plurality of sequences, *e.g*., a plurality having the same barcodes (UID). In some embodiments barcodes (UIDs) are used to determine a consensus from a plurality of sequences all having an identical barcode (UID). In other embodiments, barcodes (UIDs) are used to eliminate artifacts, *i.e.,* variations existing in some but not all sequences having an identical barcode (UID). Such artifacts resulting from PCR errors or sequencing errors can be eliminated.

In some embodiments, the number of each sequence in the sample can be quantified by quantifying relative numbers of sequences with each barcode (UID) in the sample. Each UID represents a single molecule in the original sample and counting different UIDs associated with each sequence variant can determine the fraction of each sequence in the original sample. A person skilled in the art will be able to determine the number of sequence reads necessary to determine a consensus sequence. In some embodiments, the relevant number is reads per UID ("sequence depth") necessary for an accurate quantitative result. In some embodiments, the desired depth is 5-50 reads per UID.

In some embodiments, barcodes (UIDs) are used to detect gene fusions and eliminate artifacts simulating gene fusion events. In some embodiments, sequence analysis involves a step of aligning the read of the target sequence to the known genome sequence. Each read must contain a target sequence mapping to the genome of interest and identical barcodes (UIDs) on both ends. A true gene fusion molecule would have a target sequence mapping to different regions of the target genome but identical barcodes (UIDs) on both ends. A molecule having a target sequence mapping to different regions of the target genome but having different barcodes on both ends is an artifact and not a true gene fusion molecule.

The inventors have observed such artifacts occurring at a frequency approaching or exceeding the frequency of rare gene fusion molecules present *in vivo.* Without being bound by a particular theory, the inventors hypothesized that such artifacts arise during PCR with library molecules. The extension of a universal primer may commence on one library molecule and undergo a template switch to continue on a second library molecule. The resulting fusion molecule will have binding sites for two universal primers and be amplified in subsequent cycles of PCR. Using the barcode matching according to the present invention identifies and eliminates such molecules from the sequencing data. Eliminating the artifacts allows detection of true gene fusion events with much higher sensitivity and specificity.

The invention is represented in more detail in Figures 1-8. Figure 1 depicts a single-stranded (denatured) library molecule according to the invention. Ligation of a single double-stranded adaptor to a double-stranded target molecule resulted in a double stranded circular molecule that can be denatured to yield structures depicted in Figure 1. BC are barcodes present in the adaptor. Each adaptor contains two identical barcodes. In some embodiments, different barcodes may be used. In either case, the barcode (or a combination of two barcodes) is unique among the adaptors used in the library preparation. Each library and the progeny thereof can be uniquely identified by two copies the same unique barcode or a unique combination of two barcodes. R and F are binding sites for a reverse and forward sequencing primers respectively. One of skill in the art will immediately appreciate that a single strand of nucleic acid (such as depicted in Figure 1) contains a binding site (complementary sequence) for one primer (the F primer in Figure 1), and the identical sequence to the opposite facing primer (R primer in Figure 1), while the complementary strand (not shown in Figure 1) would have identical sequence to the F primer and the binding site (complementary sequence) to the R primer. STOP is the strand synthesis terminator described further herein.

Optionally, the circular templates shown in Figure 1 may be isolated from the sample using paramagnetic beads. A non-extendable capture probe complementary to the adaptor molecule is added to the sample. Two capture probes may be used to capture each strand of the circular molecule. The capture probe is biotinylated at the 3'-end and can be captured with streptavidin-coated paramagnetic beads. The probes may have the following structure:
F'--- R' --- Biotin 3' and
R --- F --- Biotin 3'

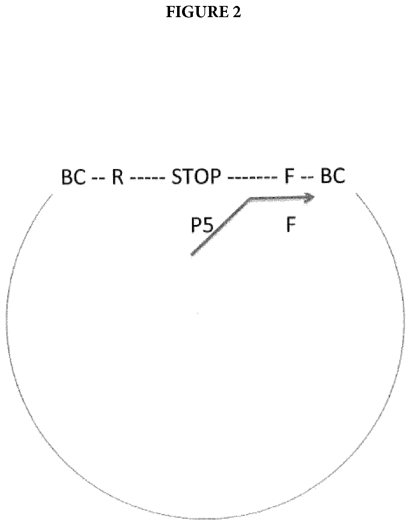

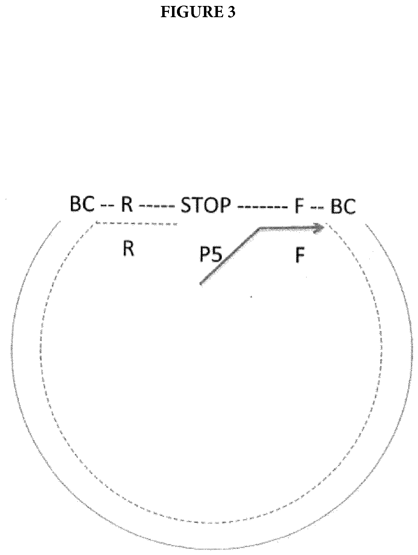

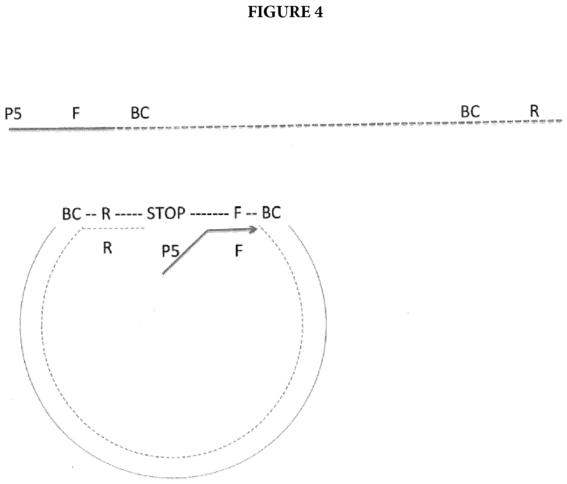

Figure 2 depicts initiation of the first strand synthesis wherein the F primer binds to the primer binding site in the library molecule. The primer has an additional non-complementary sequence at its 5'-end. The additional sequence can contain a functional element, e.g., a sequencing primer binding site (P5). Figure 3 depicts first strand synthesis and termination at the STOP. Figure 4 depicts the duplex circular molecule of Figure 3 and the separated (denatured) newly synthesized first strand the sequencing primer binding site (P5), the sequence of the forward primer (F), the target sequence flanked by two barcodes (BC) and a binding site for the reverse primer (R). STOP is not present in the newly synthesized first strand.

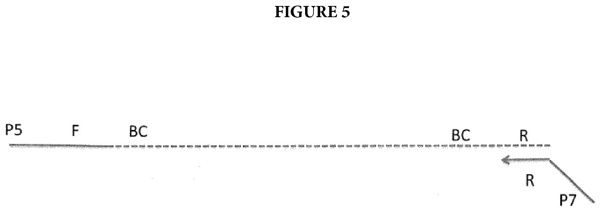

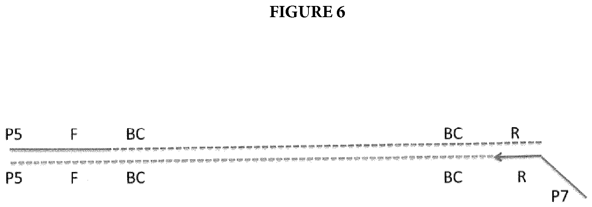

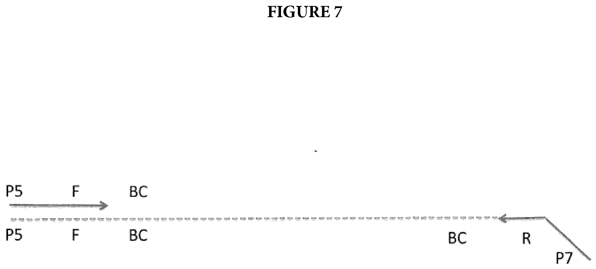

Figure 5 depicts initiation of the first strand synthesis wherein the R primer binds to the primer binding site in the first strand. The primer has an additional non-complementary sequence at its 5'-end. The additional sequence can contain a functional element, e.g., a sequencing primer binding site (P7). Figure 6 depicts second strand synthesis that copies all the elements of the first strand including the sequencing primer binding site P5. Figure 7 depicts the next round of initiation of the first strand synthesis wherein the P5 sequencing primer binds to its binding site in the second strand.

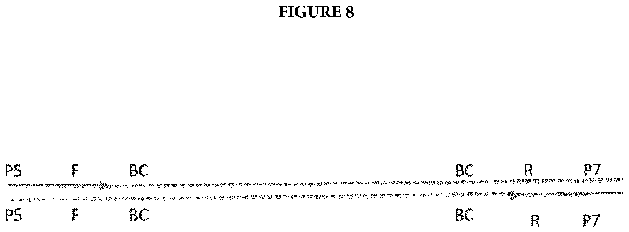

Figure 8 depicts the final linear double-stranded library molecule ready for further steps, such as amplification and sequencing. The double stranded molecule contains sequencing (or amplification) primer binding sites P5 and P7 and barcodes (BC). The molecule also retains the initial forward and reverse primer binding sites F and R. The double-stranded library molecule is characterized by a unique barcode (or a unique combination of barcodes) that distinguished this molecule and its progeny from all other molecules and their progeny in the sample.

### Example 1 (prophetic)

### Creating a library of barcoded circular molecules

In this example, DNA is isolated from the sample. The isolated DNA is optionally fragmented and size selected for an optimal size of circular molecules. The size selection step may be omitted where rare target nucleic acids are present. In some instances, RNA is isolated from the sample and reverse-transcribed into cDNA and treated in subsequent steps as DNA isolated directly from the sample.

The DNA is end-repaired and A-tailed with T4 DNA polymerase. The addition of the A-tail allows for a subsequent efficient ligation, avoiding complications from blunt ligation.

Next, a double-stranded linker is ligated to input DNA to form circular molecules. The double-stranded linker has the structure
5'P[T] -BC-R-STOP-F--BC-3'
3'BC-R'-STOP-F'-BC-[T]5'P
Where 5'P is a 5'-phosphate, [T] is the added T that base pairs with A at the 3'-end of the target molecule, BC is a barcode, STOP is the terminator nucleotide, and R and F are reverse and forward primer binding sites respectively. Optionally, the sample is treated with T7 exonuclease to remove non-circularized DNA and excess adaptors (any DNA with free ends remaining in the sample).

Optionally, the circular templates may be isolated from the sample using paramagnetic beads. Two non-extendable capture probes are used to capture each strand of the circular molecule. The capture probe is biotinylated at the 3'-end and is captured with streptavidin-coated paramagnetic beads. The capture process comprises the steps of heat denaturation, binding to beads, removal of bead-captured DNA from solution using a magnet, optional washing the beads, and elution from the bead-bound capture probes by denaturation at elevated temperature.

The isolated circular templates are then amplified by PCR. The PCR is performed with primers complementary to the primer-binding sites in the adaptor. Each primer has a 5'-flap non-complementary to the binding sites in the adaptor and containing sequencing primer binding sites or flow cell binding sequences depending on the choice of the sequencing instrument and technology. The PCR produces linear molecules. The STOP bases in each strand of the circular template molecule prevent the completion of a circle by the polymerase.

Next, the sequence data derived from the linear templates are analyzed. A molecule having a target sequence mapping to different regions of the target genome but identical (or previously matched) barcodes on both ends is detected as a true gene fusion molecule. A molecule having a target sequence mapping to different regions of the target genome but different (or not previously matched) barcodes on both ends is an artifact that is discarded from the sequencing data.

## Claims

1. A method of making a library of target nucleic acid molecules from a sample comprising a plurality of target molecules, the method comprising for substantially each target molecule:
a. ligating a single adaptor to a target molecule forming a circular molecule, wherein the adaptor comprises two barcodes, two primer binding sites situated between the two barcodes, wherein the primers annealing to the binding sites are facing away from each other, and at least one modified nucleotide effecting a strand synthesis termination by a nucleic acid polymerase situated between the two primer binding sites;
b. annealing a forward primer complementary to the adaptor to one strand of the target molecule;
c. extending the forward primer up to the modified nucleotide, thereby producing a first strand;
d. annealing a reverse primer complementary to the adaptor to the first strand;
e. extending the first primer, thereby producing the second strand and a double-stranded molecule comprising the first strand and the second strand wherein the two barcodes are flanking the target sequence.

2. The method of claim 1, wherein at least one of the forward and the reverse primer comprises a 5'-flap sequence not complementary to the adaptor and comprising an additional primer binding site.

3. The method of claim 2, further comprising a step of annealing an additional primer to the sequence complementary to the flap sequence in the forward primer and extending the additional primer thereby producing a double-stranded molecule comprising two additional primer sites and the two barcodes flanking the target sequence.

4. The method of claim 1, wherein the target molecule and the adaptor in step a. are single-stranded.

5. The method of claim 1, wherein the target molecule and the adaptor in step a. are double-stranded and the circular molecule is at least partially denatured primer to annealing of the primer in step b.

6. The method of claim 1, wherein the barcode is a nucleotide sequence 4-20 bases long.

7. The method of claim 1, wherein modified nucleotide effecting a strand synthesis termination by a nucleic acid polymerase is selected from abasic nucleotides, nucleotides with protein side groups, synthetic nucleotide AraC (cytarabine) or deoxyuracil, isoguanine, 5-methylisocytosine, ethylene glycol spacers, nucleotides with bulky analogues such as fluorophores, or unnatural base pair (UBP) "d5SICS-dNaM" nucleic acid analogues.

8. The method of claim 1, wherein ligation is selected from overhang ligation, T-A ligation, blunt-end ligation and topoisomerase-catalyzed ligation.

9. The method of claim 1, wherein the adaptor has a photocleavable linker on one end.

10. A library of target nucleic acid molecules wherein each molecule is a circular molecule comprising a target sequence and an adaptor linking the ends of the target sequence, the adaptor comprising:
a. two barcodes;
b. two primer binding sites situated between the two barcodes, wherein the primers annealing to the binding sites are facing away from each other;
c. at least one modified nucleotide effecting a strand synthesis termination by a nucleic acid polymerase situated between the two primer binding sites.

11. The library of claim 10, wherein the barcode is a nucleotide sequence 4-20 bases long.

12. The library of claim 10, wherein modified nucleotide effecting a strand synthesis termination by a nucleic acid polymerase is selected from abasic nucleotides, nucleotides with protein side groups, synthetic nucleotide AraC (cytarabine) or deoxyuracil, isoguanine, 5-methylisocytosine, ethylene glycol spacers, nucleotides with bulky analogues such as fluorophores, or unnatural base pair (UBP) "d5SICS-dNaM" nucleic acid analogues.

13. A method of sequencing target nucleic acids in a sample comprising a plurality of target molecules, the method comprising:
a. creating a library of target nucleic acid molecules from the sample by ligating a single double-stranded adaptor to substantially each double-stranded target molecule forming a double stranded circular molecule, wherein the adaptor comprises two barcodes, two primer binding sites situated between the two barcodes, wherein the primers annealing to the binding sites are facing away from each other, and at least one modified nucleotide effecting a strand synthesis termination by a nucleic acid polymerase situated between the two primer binding sites;
b. denaturing at least a portion of the double-stranded circular target molecule;
c. annealing a forward primer complementary to the adaptor to one strand of the target molecule;
d. extending the forward primer up to the modified nucleotide, thereby producing a first strand;
e. annealing a reverse primer complementary to the adaptor to the first strand;
f. extending the first primer, thereby producing the second strand and a double-stranded molecule comprising the first strand sand the second strand wherein the two barcodes are flanking the target sequence;
g. amplifying the double stranded molecule from step f.; and
h. sequencing the amplified products from step g.

14. The method of claim 13, wherein at least one of the forward and the reverse primer comprises a 5'-flap sequence not complementary to the adaptor and comprising an additional primer binding site.

15. The method of claim 14, further comprising after step f., a step f1. of annealing an additional primer to the sequence complementary to the flap sequence in the forward primer and extending the additional primer thereby producing a double-stranded molecule comprising two additional primer sites and the two barcodes flanking the target sequence.

16. The method of claim 15, wherein amplifying in step g. is performed with the additional primers.

17. The method of claim 15, wherein sequencing in step h. is performed with the additional primers.

## Patentansprüche

1. Verfahren zum Erstellen einer Bibliothek von Zielnukleinsäuremolekülen aus einer Probe, umfassend eine Vielzahl von Zielmolekülen, wobei das Verfahren für im Wesentlichen jedes Zielmolekül Folgendes umfasst:
a. Ligieren eines einzelnen Adaptors an ein Zielmolekül unter Bildung eines kreisförmigen Moleküls, wobei der Adaptor zwei Barcodes, zwei Primer-Bindungsstellen, die zwischen den beiden Barcodes liegen, wobei die Primer, die an die Bindungsstellen annealen, voneinander abgewandt sind, und mindestens ein modifiziertes Nukleotid umfasst, das eine Strangsynthesetermination durch eine Nukleinsäurepolymerase, die zwischen den beiden Primer-Bindungsstellen liegt, bewirkt;
b. Annealen eines zu dem Adaptor komplementären Vorwärts-Primers an einen Strang des Zielmoleküls;
c. Verlängern des Vorwärts-Primers bis zu dem modifizierten Nukleotid, wodurch ein erster Strang produziert wird;
d. Annealen eines zu dem Adaptor komplementären Rückwärts-Primers an den ersten Strang;
e. Verlängern des ersten Primers, wodurch der zweite Strang und ein doppelsträngiges Molekül, das den ersten Strang und den zweiten Strang umfasst, produziert werden, wobei die beiden Barcodes die Zielsequenz flankieren.

2. Verfahren nach Anspruch 1, wobei zumindest einer von dem Vorwärts- und dem Rückwärts-Primer eine 5'-FLAP-Sequenz umfasst, die zu dem Adaptor nicht komplementär ist und eine zusätzliche Primer-Bindungsstelle umfasst.

3. Verfahren nach Anspruch 2, ferner umfassend einen Schritt des Annealens eines zusätzlichen Primers an die zu der FLAP-Sequenz in dem Vorwärts-Primer komplementäre Sequenz und Verlängerns des zusätzlichen Primers, wodurch ein doppelsträngiges Molekül produziert wird, das zwei zusätzliche Primerstellen und die beiden die Zielsequenz flankierenden Barcodes umfasst.

4. Verfahren nach Anspruch 1, wobei das Zielmolekül und der Adaptor in Schritt a. einzelsträngig sind.

5. Verfahren nach Anspruch 1, wobei das Zielmolekül und der Adaptor in Schritt a. doppelsträngig sind und das kreisförmige Molekül zumindest teilweise denaturierter Primer zum Annealen des Primers in Schritt b ist.

6. Verfahren nach Anspruch 1, wobei der Barcode eine 4 - 20 Basen lange Nukleotidsequenz ist.

7. Verfahren nach Anspruch 1, wobei das modifizierte Nukleotid, das eine Strangsynthesetermination durch eine Nukleinsäurepolymerase bewirkt, aus abasischen Nukleotiden, Nukleotiden mit Proteinseitengruppen, synthetischem Nukleotid AraC (Cytarabin) oder Desoxyuracil, Isoguanin, 5-Methylisocytosin, Ethylenglykol-Spacern, Nukleotiden mit großen Analoga wie Fluorophoren oder Nukleinsäureanaloga des unnatürlichen Basenpaars (UBP) ,,d5SICS-dNaM" ausgewählt ist.

8. Verfahren nach Anspruch 1, wobei die Ligation aus Überhangligation, T-A-Ligation, Blunt-End-Ligation und Topoisomerase-katalysierter Ligation ausgewählt ist.

9. Verfahren nach Anspruch 1, wobei der Adaptor an einem Ende einen lichtspaltbaren Linker aufweist.

10. Bibliothek von Zielnukleinsäuremolekülen, wobei jedes Molekül ein kreisförmiges Molekül ist, das eine Zielsequenz und einen Adaptor, der die Enden der Zielsequenz verknüpft, umfasst, wobei der Adaptor Folgendes umfasst:
a. zwei Barcodes;
b. zwei Primer-Bindungsstellen, die zwischen den beiden Barcodes liegen, wobei die Primer, die an die Bindungsstellen annealen, voneinander abgewandt sind;
c. mindestens ein modifiziertes Nukleotid, das eine Strangsynthesetermination durch eine Nukleinsäurepolymerase, die zwischen den beiden Primer-Bindungsstellen liegt, bewirkt.

11. Bibliothek nach Anspruch 10, wobei der Barcode eine 4 - 20 Basen lange Nukleotidsequenz ist.

12. Bibliothek nach Anspruch 10, wobei das modifizierte Nukleotid, das eine Strangsynthesetermination durch eine Nukleinsäurepolymerase bewirkt, aus abasischen Nukleotiden, Nukleotiden mit Proteinseitengruppen, synthetischem Nukleotid AraC (Cytarabin) oder Desoxyuracil, Isoguanin, 5-Methylisocytosin, Ethylenglykol-Spacern, Nukleotiden mit großen Analoga wie Fluorophoren oder Nukleinsäureanaloga des unnatürlichen Basenpaars (UBP) "dSSICS-dNaM" ausgewählt ist.

13. Verfahren zum Sequenzieren von Zielnukleinsäuren in einer Probe, umfassend eine Vielzahl von Zielmolekülen, wobei das Verfahren Folgendes umfasst:
a. Erstellen einer Bibliothek von Zielnukleinsäuremolekülen aus der Probe durch Ligieren eines einzelnen doppelsträngigen Adaptors an im Wesentlichen jedes doppelsträngige Zielmolekül unter Bildung eines doppelsträngigen kreisförmigen Moleküls, wobei der Adaptor zwei Barcodes, zwei Primer-Bindungsstellen, die zwischen den beiden Barcodes liegen, wobei die Primer, die an die Bindungsstellen annealen, voneinander abgewandt sind, und mindestens ein modifiziertes Nukleotid umfasst, das eine Strangsynthesetermination durch eine Nukleinsäurepolymerase, die zwischen den beiden Primer-Bindungsstellen liegt, bewirkt;
b. Denaturieren zumindest eines Abschnitts des doppelsträngigen kreisförmigen Zielmoleküls;
c. Annealen eines zu dem Adaptor komplementären Vorwärts-Primers an einen Strang des Zielmoleküls;
d. Verlängern des Vorwärts-Primers bis zu dem modifizierten Nukleotid, wodurch ein erster Strang produziert wird;
e. Annealen eines zu dem Adaptor komplementären Rückwärts-Primers an den ersten Strang;
f. Verlängern des ersten Primers, wodurch der zweite Strang und ein doppelsträngiges Molekül, das den ersten Strang und den zweiten Strang umfasst, produziert werden, wobei die beiden Barcodes die Zielsequenz flankieren;
g. Amplifizieren des doppelsträngigen Moleküls aus Schritt f. und
h. Sequenzieren der amplifizierten Produkte aus Schritt g.

14. Verfahren nach Anspruch 13, wobei zumindest einer von dem Vorwärts- und dem Rückwärts-Primer eine 5'-FLAP-Sequenz umfasst, die zu dem Adaptor nicht komplementär ist und eine zusätzliche Primer-Bindungsstelle umfasst.

15. Verfahren nach Anspruch 14, ferner umfassend nach Schritt f. einen Schritt fl. des Annealens eines zusätzlichen Primers an die zu der FLAP-Sequenz in dem Vorwärts-Primer komplementäre Sequenz und Verlängerns des zusätzlichen Primers, wodurch ein doppelsträngiges Molekül produziert wird, das zwei zusätzliche Primerstellen und die beiden die Zielsequenz flankierenden Barcodes umfasst.

16. Verfahren nach Anspruch 15, wobei das Amplifizieren in Schritt g. mit den zusätzlichen Primern durchgeführt wird.

17. Verfahren nach Anspruch 15, wobei das Sequenzieren in Schritt h. mit den zusätzlichen Primern durchgeführt wird.

## Revendications

1. Procédé de fabrication d'une bibliothèque de molécules d'acides nucléiques cibles à partir d'un échantillon comprenant une pluralité de molécules cibles, le procédé comprenant pour sensiblement chaque molécule cible :
a. la ligature d'un unique adaptateur à une molécule cible formant une molécule circulaire, dans lequel l'adaptateur comprend deux codes-barres, deux sites de liaison d'amorce situés entre les deux codes-barres, dans lequel les amorces s'hybridant aux sites de liaison sont opposées l'une à l'autre, et au moins un nucléotide modifié réalisant une terminaison de synthèse de brin par une polymérase d'acide nucléique située entre les deux sites de liaison d'amorce ;
b. l'hybridation d'une amorce sens complémentaire à l'adaptateur à un brin de la molécule cible ;
c. l'extension de l'amorce sens jusqu'au nucléotide modifié, produisant ainsi un premier brin ;
d. l'hybridation d'une amorce antisens complémentaire à l'adaptateur au premier brin ;
e. l'extension de la première amorce, produisant ainsi le second brin et une molécule double brin comprenant le premier brin et le second brin dans lequel les deux codes-barres flanquent la séquence cible.

2. Procédé selon la revendication 1, dans lequel au moins l'une des amorces sens et antisens comprend une séquence 5'-flap non complémentaire à l'adaptateur et comprenant un site de liaison d'amorce supplémentaire.

3. Procédé selon la revendication 2, comprenant en outre une étape d'hybridation d'une amorce supplémentaire à la séquence complémentaire à la séquence flap dans l'amorce sens et d'extension de l'amorce supplémentaire produisant ainsi une molécule double brin comprenant deux sites d'amorce supplémentaires et les deux codes-barres flanquant la séquence cible.

4. Procédé selon la revendication 1, dans lequel la molécule cible et l'adaptateur dans l'étape a. sont simple brin.

5. Procédé selon la revendication 1, dans lequel la molécule cible et l'adaptateur dans l'étape a. sont double brin et la molécule circulaire est une amorce au moins partiellement dénaturée pour l'hybridation de l'amorce dans l'étape b.

6. Procédé selon la revendication 1, dans lequel le code-barres est une séquence nucléotidique longue de 4 à 20 bases.

7. Procédé selon la revendication 1, dans lequel le nucléotide modifié réalisant une terminaison de synthèse de brin par une polymérase d'acide nucléique est choisi parmi les nucléotides abasiques, les nucléotides dotés de groupes latéraux de protéines, le nucléotide synthétique AraC (cytarabine) ou le désoxyuracile, l'isoguanine, la 5-méthylisocytosine, les espaceurs d'éthylène glycol, les nucléotides dotés d'analogues volumineux tels que les fluorophores ou des analogues d'acides nucléiques de type paires de bases non naturelles (UBP) « d5SICS-dNaM ».

8. Procédé selon la revendication 1, dans lequel la ligature est choisie parmi une ligature d'extrémité protubérante, une ligature T-A, une ligature d'extrémité franche et une ligature catalysée par la topoisomérase.

9. Procédé selon la revendication 1, dans lequel l'adaptateur a un lieur photoclivable sur une extrémité.

10. Bibliothèque de molécules d'acides nucléiques cibles dans laquelle chaque molécule est une molécule circulaire comprenant une séquence cible et un adaptateur liant les extrémités de la séquence cible, l'adaptateur comprenant :
a. deux codes-barres ;
b. deux sites de liaison d'amorce situés entre les deux codes-barres, dans laquelle les amorces s'hybridant aux sites de liaison sont opposées l'une à l'autre ;
c. au moins un nucléotide modifié réalisant une terminaison de synthèse de brin par une polymérase d'acide nucléique située entre les deux sites de liaison d'amorce.

11. Bibliothèque selon la revendication 10, dans laquelle le code-barre est une séquence nucléotidique longue de 4 à 20 bases.

12. Bibliothèque selon la revendication 10, dans laquelle le nucléotide modifié réalisant une terminaison de synthèse de brin par une polymérase d'acide nucléique est choisi parmi les nucléotides abasiques, les nucléotides dotés de groupes latéraux de protéines, le nucléotide synthétique AraC (cytarabine) ou le désoxyuracile, l'isoguanine, la 5-méthylisocytosine, les espaceurs d'éthylène glycol, les nucléotides dotés d'analogues volumineux tels que les fluorophores ou des analogues d'acides nucléiques de type paires de bases non naturelles (UBP) « d5SICS-dNaM ».

13. Procédé de séquençage d'acides nucléiques cibles dans un échantillon comprenant une pluralité de molécules cibles, le procédé comprenant :
a. la création d'une bibliothèque de molécules d'acides nucléiques cibles à partir de l'échantillon par ligature d'un unique adaptateur double brin à sensiblement chaque molécule cible double brin formant une molécule circulaire double brin, dans lequel l'adaptateur comprend deux codes-barres, deux sites de liaison d'amorce situés entre les deux codes-barres, dans lequel les amorces s'hybridant aux sites de liaison sont opposées l'une à l'autre, et au moins un nucléotide modifié réalisant une terminaison de synthèse de brin par une polymérase d'acide nucléique située entre les deux sites de liaison d'amorce ;
b. la dénaturation d'au moins une partie de la molécule cible circulaire double brin ;
c. l'hybridation d'une amorce sens complémentaire à l'adaptateur à un brin de la molécule cible ;
d. l'extension de l'amorce sens jusqu'au nucléotide modifié, produisant ainsi un premier brin ;
e. l'hybridation d'une amorce antisens complémentaire à l'adaptateur au premier brin ;
f. l'extension de la première amorce, produisant ainsi le second brin et une molécule double brin comprenant le premier brin et le second brin dans lequel les deux codes-barres flanquent la séquence cible ;
g. l'amplification de la molécule double brin de l'étape f. ; et
h. le séquençage des produits amplifiés de l'étape g.

14. Procédé selon la revendication 13, dans lequel au moins l'une des amorces sens et antisens comprend une séquence 5'-flap non complémentaire à l'adaptateur et comprenant un site de liaison d'amorce supplémentaire.

15. Procédé selon la revendication 14, comprenant en outre après l'étape f., une étape fl. d'hybridation d'une amorce supplémentaire à la séquence complémentaire à la séquence flap dans l'amorce sens et d'extension de l'amorce supplémentaire produisant ainsi une molécule double brin comprenant deux sites d'amorce supplémentaires et les deux codes-barres flanquant la séquence cible.

16. Procédé selon la revendication 15, dans lequel l'amplification à l'étape g. est effectuée avec des amorces supplémentaires.

17. Procédé selon la revendication 15, dans lequel le séquençage à l'étape h. est effectué avec des amorces supplémentaires.
